# EUROPEAN PATENT APPLICATION

(11) **EP 4 556 014 A1**
(43) Date of publication of application: **21.05.2025**
(21) Application number: 24194945.2
(22) Date of filing: 16.08.2024
(51) Int. Cl.: A61K 36/068, A61K 31/716, A61K 36/07, A61K 36/074, A61P 25/28

(54) **APPLICATION OF PURIFIED MUSHROOM BETA GLUCAN IN PREVENTING, IMPROVING OR TREATING ALZHEIMER'S DISEASE, DEMENTIA OR BRAIN FUNCTION DEGENERATION**

(30) Priority: 17.11.2023 TW 112144606
(71) Applicant: Chen, Shiu-Nan, Taipei City 114 (TW)
(72) Inventor: CHEN, Shiu-Nan, 114 Taipei City (TW); CHIU, Chih-Yung, 114 Taipei City (TW)
(74) Representative: Gill Jennings & Every LLP

(57) **Abstract**

Provided is a method for preventing, improving or treating Alzheimer's disease, dementia or brain function degeneration in a subject in need, including administering to the subject a composition comprising purified mushroom β-glucan, wherein the purified β-glucan is derived from mushroom mycelium or its fermentation product, wherein the purity of the purified mushroom β-glucan is 60% or above.

## Description

### BACKGROUND

### 1. Technical Field

The present disclosure relates to use of purified mushroom β-glucan for the manufacture of a composition for the prevention, improvement or treatment of Alzheimer's disease, dementia or brain function degeneration, and in particular, relates to mushroom β-glucan purified from various mushroom mycelium or its fermentation product.

### 2. Description of Related Art

Dementia is a general term used to describe a variety of symptoms, mainly referring to a decline in memory or other thinking abilities that is severe enough to adversely affect daily activities. It has been reported that Alzheimer's Disease is the most common type of dementia and is the cause of approximately 60 to 80% of cases of dementia, much higher than Parkinson's disease or vascular dementia that accompanies stroke.

Alzheimer's disease is a neurodegenerative disease in which neurons in the hippocampus are destroyed. From the pathology, it can be found that brain tissue shrinks and amyloid β plaques, senile plaques or neurofibrillary tangles appear in the cortex. It has been reported that in patients with Alzheimer's disease, the concentration of amyloid β inside and outside brain cells is too high, leading brain cells have difficulty staying healthy and communicating with each other. Hippocampus in the brain area has a major role in learning and memory. Brain cells in this area tend to be damaged first, so one of the earliest symptoms of Alzheimer's disease is usually memory loss. As the condition worsens, patients will have problems recognizing time, space, and people, as well as develop a variety of cognitive dysfunctions.

In addition to humans, elderly companion animals, including dogs, cats, etc., may also suffer from dementia, and this disease in canines and felines is called cognitive dysfunction syndrome (CDS). CDS in elderly pets has become one of the most troublesome problems in pet care. According to statistics, 1/3 of dogs over 11 years old suffer from CDS, and nearly 70% of dogs over 15 years old have CDS. In addition, the prevalence of CDS among cats over 15 years old exceeds 50%. CDS is pathologically a progressive cognitive degeneration, and its clinical symptoms are mainly behavioral changes. Both pathologically and clinically, CDS is similar to Alzheimer's disease in humans. CDS in elderly pets not only seriously affects pets' health, but also increases the caregiving problems for owners.

Fungi mushrooms including *Ganoderma lucidum*, *Schizophyllum commune*, *Agaricus blazei*, *Cordyceps sinensis*, *Coriolus versicolor*, *Taiwanofungus camphoratus*, *Phellinus linteus*, *Hericium erinaceus*, *Auricularia auricula*, *Cordyceps militaris* etc. have been considered to be medicinal materials with a variety of excellent effects in the field of traditional Chinese medicine for years. Recently, many scientific studies have found that these various mushrooms have kinds of effects, such as improving immunity, anti-tumor, slowing down cancer metastasis, regulating physiological functions, improving cardiovascular diseases, repairing cell tissues, anti-aging, and improving cognitive impairment. Mushrooms have complex and diverse β-glucans, and many studies have found that a variety of mushroom polysaccharides among them have high activities and remarkable medical effects on diseases that are currently difficult to cure, including cancer, cardiovascular diseases, metabolic diseases, etc.

At present, many studies on Alzheimer's disease or dementia are conducted with crude extracts of mushroom fruiting bodies, spores, mycelium or their liquid fermentation products. Although it has been shown to have preventive or improving effects, the crude extract used contains a variety of polysaccharides, glycoproteins, organic acids and complex peptide molecules, and it is impossible to effectively confirm the active ingredients that truly have effects. On the other hand, due to the low yield of mushroom β-glucan, repeated and cumbersome purification techniques are required to remove impurities such as glycoproteins and small molecule peptides in order to obtain trace amounts of high-purity mushroom β-glucan. Therefore, there is currently no studies on the use of β-glucan from medicinal mushrooms such as *Ganoderma lucidum*, *Schizophyllum commune, Taiwanofungus camphoratus*, *Hericium erinaceus*, *Agaricus blazei*, *Cordyceps sinensis, Cordyceps militaris, Phellinus linteus, Coriolus versicolor, Auricularia auricula* and the like for the prevention, improvement or treatment of Alzheimer's disease or dementia.

Accordingly, there remains an unmet need to develop mushroom β-glucan with high purity to treat or prevent such conditions.

### SUMMARY

In view of the above, the present disclosure provides use of purified mushroom β-glucan for the manufacture of a composition for the prevention, improvement or treatment of Alzheimer's disease, dementia or brain function degeneration, wherein the purified mushroom β-glucan is derived from mushroom mycelium or its fermentation product, wherein the purity of the purified mushroom β -glucan is 60% or above.

The present disclosure also provides a composition comprising purified mushroom β-glucanfor use in preventing, improving or treating Alzheimer's disease, dementia or brain function degeneration in a subject in need thereof, wherein the purified mushroom β-glucan is derived from mushroom mycelium or its fermentation product and wherein the purity of the purified mushroom β-glucan is 60% or above.

In some embodiments, the mushroom of the mushroom mycelium is at least one selected from the group consisting of *Ganoderma lucidum, Schizophyllum commune*, *Agaricus blazei*, *Cordyceps sinensis*, *Coriolus versicolor*, *Taiwanofungus camphoratus*, *Phellinus linteus*, *Hericium erinaceus*, *Auricularia auricula* and *Cordyceps militaris.*

In some embodiments, the prevention, improvement or treatment of Alzheimer's disease, dementia or brain function degeneration comprises increasing the viability of neurons by purified mushroom β-glucan.

In some embodiments, the prevention, improvement or treatment of Alzheimer's disease, dementia or brain function degeneration comprises decreasing oxidative stress of neurons by purified mushroom β-glucan.

In some embodiments, decreasing oxidative stress of neurons comprises at least one of decreasing the level of inducible nitric oxide synthase, increasing the activity of catalase and/or superoxide dismutase, and decreasing the activity of lactate dehydrogenase in the subject.

In some embodiments, the prevention, improvement or treatment of Alzheimer's disease, dementia or brain function degeneration comprises decreasing the cytotoxicity to neurons caused by amyloid (amyloid β (1-40)) and/or decreasing amyloid accumulation by purified mushroom β-glucan.

In some embodiments, the prevention, improvement or treatment of Alzheimer's disease, dementia or brain function degeneration comprises preventing, improving or treating cognitive dysfunction by purified mushroom β-glucan, wherein cognitive dysfunction comprises at least one of memory impairment, memory loss, learning impairment and loss of spatial perception.

In some embodiments, purified mushroom β-glucan is obtained by the following steps, comprising:
taking fermented mushroom mycelium and fermentation broth after liquid fermentation and then obtaining crude extract after ultrasonic disruption;
centrifuging crude extract and taking the supernatant;
performing a first filtration on the supernatant with a 0.8 to 3 µm filter membrane and then a 0.22 to 0.5 µm filter membrane to filter the supernatant;
performing a first freeze-drying to prepare mushroom polysaccharide raw material;
redissolving mushroom polysaccharide raw material in water to form a redissolved liquid;
performing a second filtration on redissolved liquid with an ultrafiltration ceramic membrane filter column with a molecular weight cutoff (MWCO) from 80 to 150KD and then a MWCO from 2 to 10KD to remove impurities;
performing a third filtration with a 0.22 µm filter membrane; and
performing a second freeze-drying to obtain purified mushroom β -glucan.

In some embodiments, a composition is in the form of powder, tablet, suppository, microcapsule, liquid, spray or insert.

In some embodiments, a composition further comprises one or more excipients. In some embodiments, the composition is obtained by mixing purified and extracted β-glucan and excipients and then drying. In some embodiments, the drying comprises spray drying or freeze drying.

In some embodiments, excipients are at least one selected from the group consisting of lactose, sucrose, glucose, fructooligosaccharides, starch, starch derivatives and dietary fiber.

In some embodiments, a composition is obtained by mixing purified mushroom β-glucan with a solvent or buffer. In some embodiments, solvent is water and buffer is physiological saline.

In some embodiments, a composition is a pharmaceutical, feed, feed additive, beverage, nutritional supplement, dairy product, food, food additive, health food raw material or health food finished product.

In some embodiments, a composition further comprises an additive, wherein an additive is at least one selected from the group consisting of a carrier, excipients, preservative, diluent, filler, absorption enhancer, sweetener and adjuvant.

In some embodiments, a composition is a pharmaceutical, comprising purified mushroom β-glucan and its pharmaceutically acceptable carrier.

In some embodiments, a pharmaceutical is administered to the subject at an effective amount of 0.04 to 1 mg/kg.bw.

In some embodiments, a pharmaceutical is administered orally, intrarectally, transdermally, transmucosally, or by injection.

Based on the above invention, the present disclosure provides mushroom β-glucan derived from purified and extracted mushroom mycelium or its fermentation product, and further applies it to prepare a composition for the prevention, improvement or treatment of Alzheimer's disease, dementia or brain function degeneration.

### BRIEF DESCRIPTIONS OF THE DRAWINGS

The above and other objects, features and effects of the present invention will be more easily understood by referring to the following description in conjunction with the accompanying drawings.
FIG. 1 shows in one embodiment of the present invention, the structure of purified mushroom β-glucan obtained by combining prior art and an improved purification process.
FIG. 2 shows in one embodiment of the present invention, the HPLC chromatogram and the molecular weight of β-glucan purified from *Ganoderma lucidum.*
FIG. 3 shows in one embodiment of the present invention, the HPLC chromatogram and the molecular weight of β-glucan purified from *Schizophyllum commune.*
FIG. 4 shows in one embodiment of the present invention, the schedule of the memory and learning test in Alzheimer's disease rats with different dosages of oral or injected GL (purified *Ganoderma lucidum* β-glucan) and SC (purified *Schizophyllum commune* β-glucan).
FIG. 5 shows in one embodiment of the present invention, the effects of oral or injected GL (purified *Ganoderma lucidum* β-glucan) and SC (purified *Schizophyllum commune* β-glucan) with different dosages on acetylcholinesterase activity in the blood of Alzheimer's disease rats.
FIG. 6 shows in one embodiment of the present invention, the effects of oral or injected GL (purified *Ganoderma lucidum* β-glucan) and SC (purified *Schizophyllum commune* β-glucan) with different dosages on the total antioxidant capacity in the blood of Alzheimer's disease rats.
FIG. 7 shows in one embodiment of the present invention, the effects of oral or injected GL (purified *Ganoderma lucidum* β-glucan) and SC (purified *Schizophyllum commune* β-glucan) with different dosages on the ability to remove nitric oxide in the blood of Alzheimer's disease rats.
FIG. 8 shows in one embodiment of the present invention, the effects of oral or injected GL (purified *Ganoderma lucidum* β-glucan) and SC (purified *Schizophyllum commune* β-glucan) with different dosages on catalase activity in the blood of Alzheimer's disease rats.
FIG. 9 shows in one embodiment of the present invention, the effects of oral or injected GL (purified *Ganoderma lucidum* β-glucan) and SC (purified *Schizophyllum commune* β-glucan) with different dosages on superoxide dismutase activity in the blood of Alzheimer's disease rats.
FIG. 10 shows in one embodiment of the present invention, the effects of oral or injected GL (purified *Ganoderma lucidum* β-glucan) and SC (purified *Schizophyllum commune* β-glucan) with different dosages on lactate dehydrogenase activity in the blood of Alzheimer's disease rats.

### DETAILED DESCRIPTIONS

Unless otherwise defined, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All public documents mentioned herein are incorporated by reference.

As used in this disclosure, the singular forms "a", "an" and "the" include plural referents unless expressly limited to one referent. The term "or" is used interchangeably with the term "and/or" unless the context clearly indicates otherwise. Terms such as "first," "second," and "third" do not specifically include the meaning of relative order, but are used for the purpose of distinction.

As used herein, the terms "comprising," "including," "having," "containing" and any other variations thereof are meant to cover a non-exclusive inclusion. For example, when describing an object as "containing" a constraint, unless otherwise stated, other components, elements, components, structures, regions, parts, devices, systems, steps or connections, etc. may also be included and other limitations shall not be excluded.

As used herein, the term "effective amount" refers to the amount of active ingredient that achieves a clinical result when the composition is administered to an individual. For example, when a composition comprising the purified mushroom β-glucan of the present disclosure is administered to an individual with Alzheimer's disease, "clinical outcomes" include reduced amyloid β (1- 40), increased total antioxidant capacity, alleviated symptoms associated with Alzheimer's disease and/or increased individual's lifespan. As recognized by those skilled in the art, effective amounts may vary depending on the route of administration, use of excipients, the possibilities of combination with other treatments, and the condition to be treated.

According to the present disclosure, the terms "improving," "treating," and the like used herein generally refers to obtaining desired pharmacological or physiological effects. The effects may be prophylactic insofar as it "prevents" the condition, appearance, disease or symptoms in whole or in part, and/or the effects may be therapeutic insofar as partial or complete cure of the condition and/or adverse effects attributable to the condition or disease. The term "treating" as used herein encompasses any treatment of a condition, disease or abnormal behavior in a mammal (e.g. a human) and includes: (a) preventing diseases (e.g., Alzheimer's disease), conditions (e.g., memory impairment), or abnormal behaviors (e.g., loss of spatial perception) from a subject who may be susceptible but have not yet been observed or diagnosed with the diseases; (b) inhibiting diseases, conditions or symptoms, i.e. causing regression of the conditions or symptoms; and (c) alleviating diseases, conditions or symptoms, i.e. causing regression of the conditions or symptoms.

As used herein, the terms "individual," "patient," and "subject" are used interchangeably. The term "subject" refers to a human or an animal. Examples of subjects include but are not limited to humans, monkeys, mice, rats, marmots, ferrets, rabbits, hamsters, cows, horses, pigs, deer, dogs, cats, foxes, wolves, chickens, emu, ostriches and fish.

As used herein, the term "administering" refers to a means or a route of introducing an active ingredient, at least in part, into the body of a subject through a specific site to produce the desired effects. The active ingredients described herein may be administered through any suitable route known in the art. For example, the pharmaceutical compositions of the present disclosure are administered to a subject orally.

As used herein, the terms "increasing," "enhancing," "promoting," or similar terms, refer to increasing or prolonging the intended efficacy or duration of action of drugs. Therefore, " 'enhancing' the efficacy of therapeutic drugs" refers to the increased or prolonged ability in the relevant treatment system, i.e., the efficacy or duration of action of drugs.

Fungus mushrooms are medicinal materials with various excellent effects, and in recent years, many studies have found that different mushrooms have the effects of improving immunity, anti-tumor, slowing down metastasis, regulating physiological functions, improving cardiovascular diseases, repairing cells and tissues, anti-aging, improving cognitive impairment, and the like. In this regard, β-glucan contained in mushrooms has been proven to have excellent effects on improving immunity and regulating physiological functions, and has been widely used in medical and health food products. Studies have found that β-glucans from different sources have different functions due to different receptors on the cell membrane and different signal transduction pathways. Mushroom β-glucans are complex and diverse. Many studies also have found that different mushroom β-glucans not only have high functional activities, but also have remarkable medical effects on many diseases that are difficult to solve by current medicine technology, such as cancer, cardiovascular diseases, metabolic diseases, etc.

Due to the low yield of mushroom β-glucan, repeated and cumbersome purification are required to remove impurities such as glycoproteins and small molecule peptides in order to obtain trace amounts of purified mushroom β-glucan. Therefore, there is currently no studies on the use of β-glucans from *Ganoderma lucidum*, *Schizophyllum commune*, *Taiwanofungus camphoratus*, *Hericium erinaceus*, *Agaricus blazei, Cordyceps sinensis, Cordyceps militaris, Phellinus linteus, Coriolus versicolor*, *Auricularia auricula*, and other medicinal mushrooms to prevent, improve, or treat Alzheimer's disease or dementia. Accordingly, the inventor of the present disclosure integrated the prior art, broke through the bottleneck in production, and then obtained purified mushroom β-glucan. Such purified mushroom β-glucan is applied in research to prevent, improve or treat Alzheimer's disease, dementia or brain function degeneration, and unexpected effects are also found.

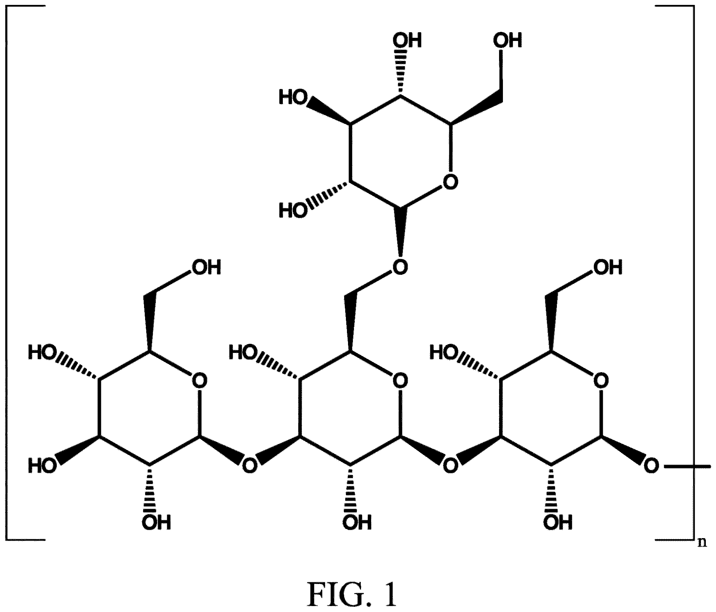

In an embodiment of the present disclosure, provided is use of purified mushroom β-glucan for the manufacture of a composition for the prevention , improvement or treatment of Alzheimer's disease, dementia or brain function degeneration. The basic unit of purified mushroom β-glucan is glucose. Purified mushroom β-glucan is composed of β 1-3 glycosidic bonds as the main chain and β 1-6 glycosidic bonds as side branches. The structure of mushroom β-glucan is shown in Fig. 1, which chemical formula is (C₂₄H₄₂O₂₁)ₙ.

In an embodiment of the present disclosure, various processes of purification and extraction can be applied and combined to achieve the goal of effectively purifying mushroom β-glucan, for example, the goal of making a purity of mushroom β-glucan to 60% or above. In addition, a purity of purified mushroom β-glucan can be freely adjusted as needed, for example, a purity of mushroom β-glucan can be from 60% to 98%, from 70% to 98%, and from 80% to 98%, such as a purity of 60%, 65%, 70%, 75%, 80%, 85%, 90%, 95%, or 98%.

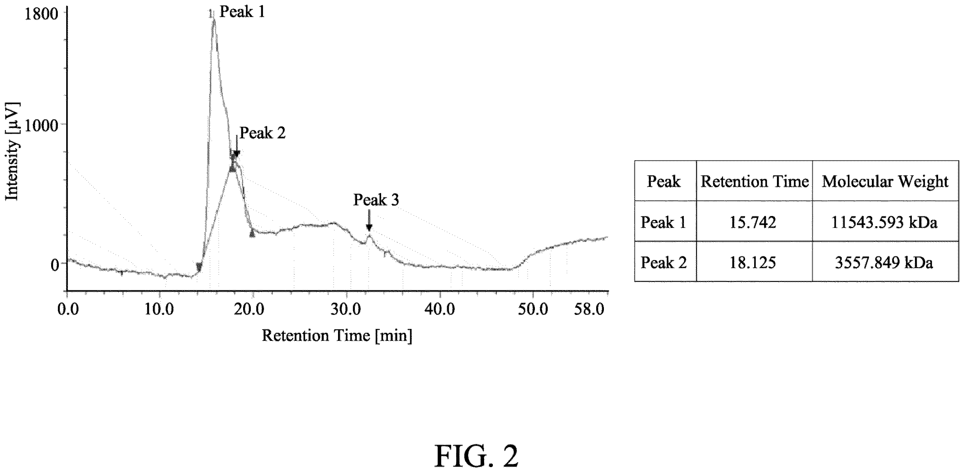

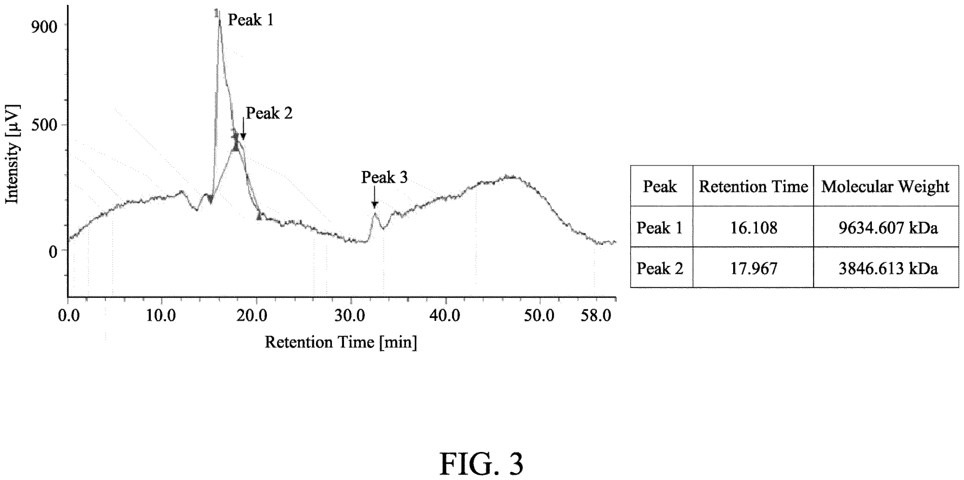

In another embodiment of the present disclosure, the molecular weight of purified mushroom β-glucan is from 3000 kDa (± 15%) to 12000 kDa (± 30%). For example, the molecular weight of β-glucan of *Ganoderma lucidum* can range from 3558 kDa to 11544 kDa (as shown in FIG. 2), and the molecular weight of *Schizophyllum commune* β-glucan can range from 3847 kDa to 9635 kDa (as shown in FIG. 3).

In another embodiment of the present disclosure, provided is also a method for preventing, improving or treating Alzheimer's disease, dementia or brain function degeneration in a subject in need, comprising administering to the subject a composition comprising purified mushroom β-glucan with a purity higher than 60%, wherein the purified mushroom β-glucan is derived from mushroom mycelium or its fermentation product.

In an embodiment of the present disclosure, the purified mushroom β-glucan is derived from mushroom mycelium or its fermentation product. In an embodiment, the mushroom of the mushroom mycelium is at least one selected from the group consisting of *Ganoderma lucidum*, *Schizophyllum commune*, *Agaricus blazei*, *Cordyceps sinensis*, *Coriolus versicolor*, *Taiwanofungus camphoratus*, *Phellinus linteus*, *Hericium erinaceus*, *Auricularia auricula* and *Cordyceps militaris.*

It has been confirmed that excessive oxidative stress is an important factor in accelerating aging. Excessive accumulation of oxides will lead to oxidative neurotoxicity, causing neuron damage, death or glial cell abnormalities and thereby resulting in the onset of neurodegenerative diseases. Therefore, decreasing oxidative stress of neurons or improving the antioxidant capacity of neurons is considered to be one of the means to prevent or treat Alzheimer's disease, dementia or other neurological diseases.

In an embodiment of the present disclosure, the prevention, improvement or treatment of Alzheimer's disease, dementia or brain function degeneration comprises increasing the viability of neurons by mushroom β-glucan, thereby achieving the purpose of protecting neurons. In some embodiments, the prevention, improvement or treatment of Alzheimer's disease, dementia or brain function degeneration comprises decreasing oxidative stress of neurons by purified mushroom β-glucan. In some embodiments, decreasing oxidative stress of neurons comprises at least one of decreasing the level of inducible nitric oxide synthase, increasing the activity of catalase and/or superoxide dismutase, and decreasing the activity of lactate dehydrogenase in a subject. In other words, the four states of increased catalase activity, increased superoxide dismutase activity, decreased lactate dehydrogenase activity, and decreased nitric oxide level may occur alone, or any two, three or four of them may occur at the same time.

In an embodiment of the present disclosure, the prevention, improvement or treatment of Alzheimer's disease, dementia or brain function degeneration comprises decreasing the cytotoxicity to neurons caused by amyloid (amyloid β (1-40)) and/or decreasing amyloid accumulation by purified mushroom β-glucan.

In an embodiment of the present disclosure, the prevention, improvement or treatment of Alzheimer's disease, dementia or brain function degeneration comprises preventing, improving or treating cognitive dysfunction by purified and extracted mushroom β-glucan. In some embodiments, cognitive dysfunction comprises at least one of memory impairment, memory loss, learning impairment, and loss of spatial perception.

According to the present disclosure, purified mushroom β-glucan that can prevent, improve or treat Alzheimer's disease, dementia or brain function degeneration can be purified by applying and combining various processes of purification and extraction, and the purified mushroom β-glucan achieves a purity of 60% or above, or from 60% to 98%. In one embodiment, the purification and extraction comprises:
taking fermented mushroom mycelium and fermentation broth after liquid fermentation and then obtaining crude extract after ultrasonic disruption;
removing the precipitate from the crude extract after centrifugation and taking out the supernatant;
subsequently, performing a first filtration on the supernatant, for example, using a filter membrane with a pore size of 0.8 to 3 µm, such as a filter membrane with a pore size of 0.8, 0.9, 1.0, 1.5, 2, 2.5 or 3 µm, to remove impurities, and then using a filter membrane with a pore size of 0.22 to 0.5 µm, such as a filter membrane with a pore size of 0.22, 0.25, 0.3, 0.35, 0.4, 0.45 or 0.5 µm, to filter out impurities;
then performing a first freeze-drying on the supernatant subjected to the first filtration, to prepare mushroom polysaccharide raw material;
redissolving the mushroom polysaccharide raw material in water to form a redissolved liquid;
performing a second filtration on the redissolved liquid, which allows the redissolved liquid to pass through an ultrafiltration ceramic membrane filter column wigh a molecular weight cut off (MWCO) of from 80 to 150KD, such as 80, 90, 100, 110, 120, 130, 140 or 150 KD and MWCO of from 2 to 10KD, such as 2, 3, 4, 5, 6, 7, 8, 9 or 10 KD respectively to remove macromolecule impurities and small molecule impurities;
performing a third filtration on the redissolved liquid subjected to the second filtration, which uses a mixed cellulose esters filter membrane with a pore size of 0.22 µm to filter impurities; and
finally, performing a second freeze-drying on the redissolved liquid subjected to the third filtering, to obtain a purified mushroom polysaccharide product.

In some embodiments, the above-mentioned filtration process can be repeated several times before subsequent steps are performed, so that a purity of the purified mushroom polysaccharide reaches 60% or above, or from 60% to 98%, such as 60, 65, 70, 75, 80, 85, 90, 95 or 98%, to meet the needs.

In an embodiment of the present disclosure, mushroom polysaccharide powder with a specific purity, such as 90% or above, is prepared into purified mushroom polysaccharide solutions with different concentrations according to the needs. The solutions are filtered with a 0.22 µm sterile syringe filter membrane, bottled, and then sterilized by autoclaving, for example, at 121°C for 20 minutes, in order to obtain bottled injections or oral liquids.

In an embodiment of the present disclosure, the composition of the present disclosure can be in any suitable form, including but not limited to powder, tablet, suppository, microcapsule, liquid, spray or insert.

In an embodiment of the present disclosure, the composition further comprises an excipient. In one embodiment, the composition is obtained by mixing purified mushroom β-glucan with an excipient, and then drying. The composition obtained by such a method is, for example, in the form of powder. In another embodiment, the drying is, for example, spray drying or freeze drying. In yet another embodiment, the excipient comprises lactose, sucrose, glucose, fructooligosaccharides, starch, starch derivatives or dietary fiber, but are not limited thereto.

In an embodiment of the present disclosure, the composition is obtained by mixing purified mushroom β-glucan with a solvent or buffer, and the obtained composition is, for example, in a liquid form. In another embodiment, the solvent can be water, and the buffer can be physiological saline or another pharmaceutically acceptable buffer, but is not limited thereto.

In an embodiment of the present disclosure, the composition of the present disclosure can be pharmaceutical, feed, feed additive, beverage, nutritional supplement, dairy product, food, food additive or health food raw material and finished product, but is not limited thereto. In one embodiment, the composition of the present disclosure is a pharmaceutical, which comprises purified mushroom β-glucan and a pharmaceutically acceptable carrier. In one embodiment, the pharmaceutical of the present disclosure is for human use or for animal use.

In some embodiments, the pharmaceutical is administered to a subject at an effective amount for preventing, improving or treating Alzheimer's disease, dementia or brain function degeneration, wherein the effective amount is from 0.04 mg to 1 mg per kilogram of human body weight (0.04 to 1 mg/kg.bw), such as 0.04, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95 or 1 mg/kg.bw. The effective amount for animals can be converted according to pharmacokinetics. In other embodiments, the pharmaceutical product is administered orally, intrarectally, transdermally, transmucosally or by injection, but is not limited thereto.

In an embodiment of the present disclosure, the composition of the present disclosure may include at least one additive selected from the following groups: a carrier, excipient, preservative, diluent, filler, absorption enhancer, sweetener, and adjuvant, but is not limited thereto.

Needless to say, it is believed that those skilled in the art can understand and utilize the present disclosure to the greatest extent based on the above description. Accordingly, the following examples are provided for illustrative purposes only and are not intended to limit the scope of the present disclosure in any way.

### Examples

### Preparation example: Preparation of high-purity mushroom β-glucan from mushroom mycelium

The purification process of purified mushroom β-glucan of the present invention was further improved on the basis of that of the applicant's previous patents (Patent Nos. 1481718 and 1686480), which entire content was incorporated herein by reference. Specifically, in a fermentation container, 1 liter of culture medium was prepared using sterile water as the solvent; using trehalose, mannose and glucose as carbon source, wherein the weight ratio of trehalose, mannose and glucose is 1:1:1; using 0.5% by weight of yeast extract as nitrogen source. Then, the prepared culture medium was placed together with the fermentation container into an autoclave for high-temperature and high-pressure sterilization. The sterilization conditions were 121°C for 15 minutes. After sterilization, it was cooled at room temperature, and then mycelium of *Ganoderma lucidum* was added and cultured at 30 to 100 rpm/min for about 15 days. The mushroom mycelium can produce a large amount of mushroom β-glucan in the culture medium.

The fermented mushroom mycelium and fermentation broth after liquid fermentation were collected, disrupted with ultrasonic waves, and mixed with its fermentation product to obtain a crude extract. The precipitate in the crude extract after centrifuging was discarded, and the supernatant was taken out and then filtered with a 0.8 µm filter membrane (AAWP04700, MF-Millipore^{®}) and a 0.45 µm mixed cellulose esters filter membrane (HAWP04700, MF-Millipore^{®}) to remove impurities. Afterwards, water was removed by freeze drying to prepare mushroom polysaccharide raw material.

The dried mushroom polysaccharide raw material was redissolved in water and passed through an ultrafiltration ceramic membrane filtration column with a MWCO from 80 to 150KD and a MWCO from 2 to 10KD to remove macromolecular impurities and small molecule respectively. It was then filtered with a 0.22 µm mixed cellulose esters filter membrane (GSWP04700, MF-Millipore^{®}) and freeze-dried to obtain purified mushroom β-glucan with a purity of 60 to 98%.

Purified mushroom β-glucan solution having various purity was prepared into solutions with various required concentrations. They were then filtered with a hydrophilic sterilized polysulfide syringe filter (SLGPR33RB, Millex^{®}) with a pore size of 0.22 µm and a diameter of 33 mm, bottled, and sterilized with high-pressure steam at 121°C for 20 minutes to prepare bottled injections or oral liquids.

Other mushroom mycelia such as *Ganoderma lucidum* and *Schizophyllum commune* were purified with reference to the aforementioned steps and used for the test and assay of subsequent examples.

The purity of the mushroom β-glucan obtained by this purification method can reach 60% or above after being redissolved in water, and it can further reach up to 98% (here, the upper limit of 98% in the purity test is limited by the current reference sample of reagent grade β-glucan (Beta Glucan, United States Pharmacopeia (USP) Reference Standard, Sigma-Aldrich, 1048288)) through multiple separation and purification.

### Example 1: Analysis of purified mushroom β-glucan

The basic unit of the purified mushroom β-glucan prepared according to the aforementioned preparation example is glucose. Glucose are linked to each other by β 1-3 glycosidic bonds to form a main chain and by β 1-6 glycosidic bonds to form side branches. The specific structure of the purified mushroom β-glucan is shown in Fig. 1.

High Performance Liquid Chromatography (HPLC) (HPLC Pump, JASCO; Autosampler, AS-4050) with a refractive index detector (RI detector, RI-4030), and interconnected Shodex SMGAR KS-804, KS-805, and KS-806 columns in series were used to measure different mushroom β-glucan molecular weights. The results showed that the molecular weight of purified *Ganoderma lucidum* β-glucan ranges from 3558 kDa to 11544 kDa (as shown in Fig. 2), and the molecular weight of purified *Schizophyllum commune* β-glucan ranges from 3847 kDa to 9635 kDa (as shown in Fig. 3).

Protein quantitative analysis (Bio-Rad Protein Assay Kit II (5000002EDU)) was also carried out for the β-glucan produced by the present invention, and the results showed that after three continuous tests on samples with a concentration of 1500 ppm, the average protein concentration of the purified *Ganoderma lucidum* β-glucan product was 2.031 *µ*g/mL, and the average protein concentration of the purified *Schizophyllum commune* β-glucan product was 3.665 µg/mL (as shown in Table 1).

**Table 1, protein concentration analysis of purified Ganoderma lucidum β-glucan (GL) and purified Schizophyllum commune β-glucan (SC)**

| Sample No. | Protein Concentration (µg/mL) | | | Average value (µg/mL) |
|---|---|---|---|---|
| | Test 1 | Test 2 | Test 3 | |
| SC (IV)/1500 ppm Lot 636015INJ20230407 | 3.487 | 3.655 | 3.824 | 3.655±0.168 |
| GL (IV)/1500 ppm Lot 021015INJ20230407 | 1.639 | 2.311 | 2.143 | 2.031±0.350 |

### Example 2: Effects of purified mushroom β-glucan on human neuroblastoma cell lines and mouse neuroblastoma cell lines

In order to evaluate the protective ability of purified mushroom β-glucan to neurons, a 48-hour cell viability test was performed on human neuroblastoma cell line (IMR 32) and mouse neuroblastoma cell line (Neuro 2a) using purified Ganoderma lucidum β-glucan as an representative. The results showed that purified Ganoderma lucidum β-glucan did not reduce cell viability. On the contrary, 24 hours after the addition amount reached 25 µg/mL, cell viability was significantly improved (as shown in Table 2), indicating that β-glucan has the function of protecting neurons.

**Table 2, cell viability of IMR 32 and Neuro 2a cells at 24 and 48 hours using purified Ganoderma lucidum β-glucan (GL) samples at different concentrations.**

| Amount (µg/mL) | IMR 32 Cells | | Neuro 2a Cells | |
|---|---|---|---|---|
| | 24 hr | 48 hr | 24 hr | 48 hr |
| | Cell viability (%) | | | |
| 0 | 103.4 ± 2.1 | 108.0 ± 2.1 | 94.9 ± 2.4 | 100.6 ± 3.2 |
| 12.5 | 107.9 ± 5.9 | 112.4 ± 7.3 | 102.7 ± 8.2 | 109.0 ± 8.5 |
| 25 | 120.9 ± 6.4* | 143.1 ± 8.2* | 125.5 ± 9.2* | 141.3 ± 9.5* |
| 50 | 137.3 ± 8.4* | 168.1 ± 9.6* | 149.8 ± 7.3* | 229.4 ± 7.6* |
| 100 | 164.5 ± 9.7* | 180.2 ± 6.5* | 205.1 ± 6.5* | 256.0 ± 6.4* |
| 200 | 228.2 ± 8.6* | 233.7 ± 8.2* | 230.6 ± 9.5* | 287.9 ± 7.8* |

### Example 3: Effects of purified Ganoderma lucidum β-glucan on learning, memory, spatial perception and working memory of mice suffering from Alzheimer's disease

Alzheimer's disease can cause damage to the hippocampus of the brain, adversely affecting responses to various behaviors such as learning, memory, and spatial perception. In this study, 56 Crl:SD rats were divided into 9 groups. Microsyringes were implanted in the hippocampus area of the brain of all rats, and Aβ₁₋₄₀ amyloid was injected daily to induce Alzheimer's disease. After implantation, rats were divided into the following groups:
AM group, which was a drug control group (positive control) that rats were fed Aricept, a commonly used Alzheimer's drug, daily;
GLI1 group, which rats were injected daily with 0.47 mg/kg.bw of purified *Ganoderma lucidum* β-glucan;
SCI1 group, which rats were injected daily with 0.47 mg/kg.bw of purified *Schizophyllum commune* β-glucan;
GL1, which rats were fed 0.47mg/kg.bw of purified *Ganoderma lucidum* β-glucan daily;
GL5 group, which rats were fed 2.35mg/kg.bw of purified *Ganoderma lucidum* β-glucan daily;
SC1 group, which rats were fed 0.47mg/kg.bw of purified *Schizophyllum commune* β-glucan daily;
SC5 group, which rats were fed 2.35mg/kg.bw of purified *Schizophyllum commune* β-glucan daily;
A group, which was a No-treatment control that rats received no drug after Alzheimer's disease was induced; and
C group, which was a control group (Control) that Alzheimer's disease was not induced and rats did not received any drug.

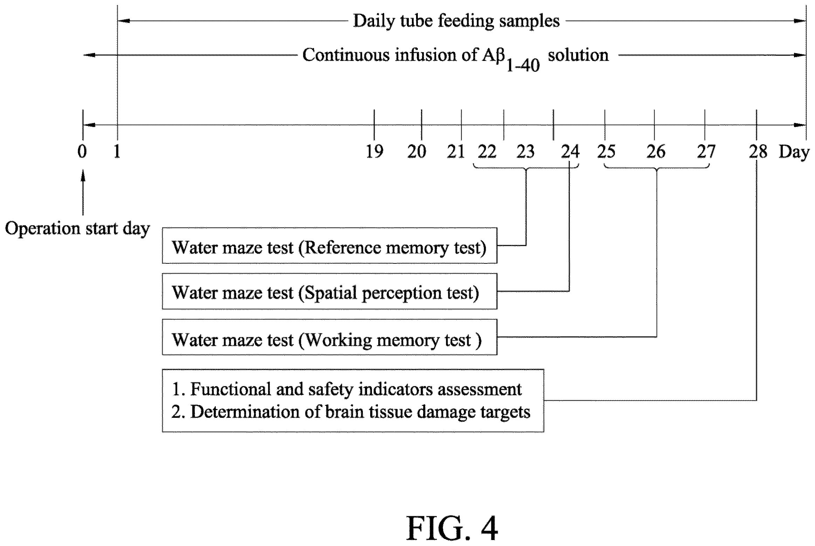

The Morris water maze (MWM) test was started 22 days after the operation, and the differences in learning and memory, spatial perception and working memory were evaluated. The detailed test schedule is shown in Fig. 4.

In the MWM test, the pool in which rats swim was separated into four quadrants to observe the rats' learning, memory and search skills. The less the swimming time and distance required to find the target, the better these abilities.

For the evaluation of learning and memory, it can be found from the data in Table 3 to Table 5 that the test groups injected with or fed purified *Ganoderma lucidum or Schizophyllum commune* β-glucan performed better than the drug control group from the first to the third day of the learning and memory test, and even far better than the control group (C group) that Alzheimer's disease was not induced and no drug was administered.

**Table 3, the retention time in each quadrant and total distance moved on the first day of the learning and memory test in rats administered GL and SC orally or by injection at different dosages.**

| Total distance moved (cm) | | Quadrant | | | |
|---|---|---|---|---|---|
| | | First Quadrant | Second Quadrant | Third Quadrant | Fourth Quadrant |
| | | Time (s) | | | |
| C | 999.2±35.1 | 14.5±4.3^{a} | 11.8±2.3 ^{a} | 12.9±3.9 ^{a} | 13.6±4.0 ^{a} |
| A | 956.6±24.8 | 14.0±4.7^{a} | 12.6±3.6^{a} | 14.8±2.4^{a} | 13.5±5.1^{a} |
| AM | 968.4±22.4 | 13.3±0.9^{a} | 10.2±0.5^{a} | 11.4±0.4^{a} | 12.8±0.4" |
| GLI1 | 985.3±13.5 | 9.1±1.4^{b} | 8.2±1.4^{a} | 9.3±1.1^{b} | 12.5±1.6^{c} |
| GL1 | 965.3±24.6 | 8.9±0.3^{a} | 9.1±0.6^{a} | 6.2±0.2^{a} | 10.8±1.4^{b} |
| GL5 | 952.4±15.8 | 7.3±1.1^{b} | 7.2±0.7^{a} | 6.3±0.3^{a} | 9.5±1.2^{c} |
| SCI1 | 975.3±19.9 | 10.7±1.1^{b} | 8.2±1.4^{a} | 10.3±1.1^{b} | 12.5±3.8^{c} |
| SC1 | 930.1±27.7 | 9.2±0.1^{b} | 6.7±0.6^{a} | 5.2±0.2^{a} | 10.8±1.4^{c} |
| SC5 | 924.2±16.3 | 8.3±1.2^{c} | 6.2±0.7^{a} | 5.3±0.3^{a} | 9.5±1.2^{c} |

| | | | | | |
|---|---|---|---|---|---|
| 1. Each value is expressed as mean ± SD (n=6). 2. The values marked ^{a,b,c} represent that the stationary platform quadrant is significantly different from other quadrants (p < 0.05). | | | | | |

**Table 4, the retention time in each quadrant and total distance moved on the second day of the learning and memory test in rats administered GL and SC orally or by injection at different dosages.**

| Total distance moved (cm) | | Quadrant | | | |
|---|---|---|---|---|---|
| | | First Quadrant | Second Quadrant | Third Quadrant | Fourth Quadrant |
| | | Time (s) | | | |
| C | 792.1±32.9 | 9.8±0.7 ^{a} | 9.4±1.1^{a} | 10.3±0.3^{a} | 12.5±0.1^{b} |
| A | 828.0±42.2 | 11.7±2.8^{a} | 11.8±2.2^{a} | 10.0±3.0^{a} | 13.5±0.6^{a} |
| AM | 798.6±36.0 | 11.4±0.8^{a} | 12.0±1.3^{a} | 10.1±0.3^{a} | 10.6±1.0^{a} |
| GLI1 | 772.0±21.4 | 12.2±0.5^{b} | 7.3±0.4^{a} | 6.1±0.4^{a} | 11.5±1.6^{b} |
| GL1 | 713.2±41.3 | 10. 2±1.2^{b} | 7.1±0.6^{a} | 5.1±1.0^{a} | 10.2±1.6^{b} |
| GL5 | 721.6±12.6 | 7.3±1.3^{b} | 6.4±0.2^{b} | 5.2±0.8^{a} | 10.6±0.7^{c} |
| SCI1 | 762.0±15.4 | 13.2±0.7^{b} | 7.6±0.7^{a} | 6.9±2.4^{a} | 11.5±1.6^{b} |
| SC1 | 717.2±50.5 | 11. 2±2.0b | 7.2±0.5^{a} | 5.3±2.0^{a} | 10.2±1.6^{b} |
| SC5 | 703.6±23.6 | 6.3±1.6" | 6.2±0.6^{a} | 5.5±1.1^{a} | 10.6±0.7^{b} |

| | | | | | |
|---|---|---|---|---|---|
| 1. Each value is expressed as mean ± SD (n=6). 2. The values marked ^{a,b,c} represent that the stationary platform quadrant is significantly different from other quadrants (p < 0.05). | | | | | |

**Table 5, the retention time in each quadrant and total distance moved on the first day of the learning and memory test in rats administered GL and SC orally or by injection at different dosages.**

| Total distance moved (cm) | | Quadrant | | | |
|---|---|---|---|---|---|
| | | First Quadrant | Second Quadrant | Third Quadrant | Fourth Quadrant |
| | | Time (s) | | | |
| C | 657.6±41.2 | 6.2±0.9^{a} | 8.7±0.4^{b} | 10.7±1.9^{c} | 8.5±0.5^{b} |
| A | 782.1±42.9 | 9.8±0.7^{b} | 7.7±1.1^{a} | 9.7±0.3^{b} | 12.7±0.8^{c} |
| AM | 743.7±33.4 | 9.0±0.7^{b} | 7.2±1.2^{a} | 8.5±0.9^{a} | 10.0±1.1^{a} |
| GLI1 | 626.0±45.2 | 5.6±1.6^{b} | 4.1±3.2^{a} | 4.4±0.8^{a} | 7.5±0.2^{b} |
| GL1 | 619.4±44.1 | 5.3±1.2^{b} | 3.6±0.7^{a} | 3.2±0.4^{a} | 7.9±0.5^{b} |
| GL5 | 602.4±38.5 | 4.5±1.3^{b} | 3.2±0.1^{a} | 3.3±1.1^{a} | 8.3±0.5^{c} |
| SCI1 | 646.3±35.1 | 5.2±0.8^{b} | 4.1±3.2^{a} | 4.5±0.6^{a} | 7.1±0.3^{c} |
| SC1 | 632.4±24.3 | 6.1±0.8^{b} | 3.6±0.7^{a} | 3.4±0.5^{a} | 7.6±0.7^{c} |
| SC5 | 623.4±28.4 | 6.5±0.7^{b} | 3.2±0.1^{a} | 3.5±0.9^{a} | 8.1±0.4^{c} |

| | | | | | |
|---|---|---|---|---|---|
| 1. Each value is expressed as mean ± SD (n=6). 2. The values marked ^{a,b,c} represent that the stationary platform quadrant is significantly different from other quadrants (p < 0.05). | | | | | |

The 90-second spatial perception test was also performed in a water maze. It was found that when rats were placed in the second quadrant and third quadrant, the test groups injected with or fed purified mushroom β-glucan presented statistical significances in the speed of space exploration compared with the C group and the AM group. The advantage was more significant in the high-dosage feeding group (GL5/SC5). Detailed data are shown in Table 6.

**Table 6, 90-second spatial perception test: the retention time in each quadrant of rats administered GL and SC orally or by injection at different dosages.**

| | First Quadrant | Second Quadrant | Third Quadrant | Fourth Quadrant |
|---|---|---|---|---|
| | Time (s) | | | |
| C | 18.3±6.1^{b} | 16.8±5.8^{b} | 16.6±6.4^{b} | 37.3±4.7^{a} |
| A | 20.3±3.8^{b} | 22.8±5.4^{b} | 18.7±6.5^{b} | 27.2±9.0^{a} |
| AM | 22.8±4.8^{b} | 14.3±3.3^{c} | 17.5±4.6^{b} | 34.3±7.0^{a} |
| GLI1 | 22.1±6.1^{b} | 17.1±7.9^{c} | 14.7±3.9^{c} | 35.1±6.9^{a} |
| GL1 | 22.7±5.3^{b} | 15.4±4.2^{c} | 15.6±4.8^{c} | 35.5±7.9^{a} |
| GL5 | 24.2±4.0^{b} | 12.5±2.3^{c} | 13.5±3.6^{c} | 37.8±5.3^{a} |
| SCI1 | 22.9±4.1^{b} | 16.8±4.2^{c} | 16.5±4.7^{c} | 32.8±7.6^{a} |
| SC1 | 26.4±3.8^{b} | 15.2±5.1^{c} | 15.4±3.6^{c} | 32.0±5.4^{a} |
| SC5 | 25.9±5.5^{b} | 13.1±4.9^{c} | 12.6±8.0^{c} | 37.4±4.6^{a} |

| | | | | |
|---|---|---|---|---|
| The values marked ^{a,b,c} represent that the stationary platform quadrant is significantly different from other quadrants (p < 0.05). | | | | |

The evaluation of working memory was carried out over three consecutive days in a water maze. From the data in Table 7 to Table 9, it can be found that the test groups fed or injected with purified mushroom β-glucan had significant advantages in the performance of working memory. From the first day, it can be seen that the high-dosage feeding group showed significantly better performance in both retention time and speed. Although the control group and the drug control group also improved working memory, overall they were still not as good as the test groups fed purified mushroom β-glucan.

**Table 7, the retention time in each quadrant and total distance moved on the first day of the working memory test in rats administered GL and SC orally or by injection at different dosages.**

| Total distance moved (cm) | | Quadrant | | | |
|---|---|---|---|---|---|
| | | First Quadrant | Second Quadrant | Third Quadrant | Fourth Quadrant |
| | | Time (s) | | | |
| C | 671.5±37.0 | 6.9±0.4^{b} | 4.5±0.7^{a} | 5.3±0.2^{a} | 6.9±0.8^{a} |
| A | 606.9±29.9 | 7.5±0.3^{a} | 6.8±1.3^{a} | 6.4±0.5^{a} | 6.6±1.5^{a} |
| AM | 553.1±12.1 | 7.1±1.4^{b} | 5.4±0.3^{a} | 5.8±1.1^{b} | 7.3±1.8^{b} |
| GLI1 | 542.4±15.5 | 7.6±0.9^{b} | 4.3±1.8^{a} | 4.1±1.0^{a} | 6.7±0.8^{b} |
| GL1 | 496.3±12.5 | 7.7±0.4^{c} | 3.2±0.7^{a} | 2.8±0.8^{a} | 6.4±0.9^{b} |
| GL5 | 476.2±13.3 | 7.8±0.3^{c} | 2.4±0.3^{a} | 2.6±0.2^{a} | 5.7±0.4^{b} |
| SCI1 | 556.5±16.3 | 7.9±0.2^{b} | 4.3±0.8^{a} | 4.7±0.7^{a} | 7.2±0.3^{b} |
| SC1 | 516.4±13.4 | 7.4±0.3^{b} | 3.5±0.4^{a} | 2.8±0.8^{a} | 7.4±0.9^{b} |
| SC5 | 496.3±14.7 | 7.5±0.3^{c} | 3.4±0.3^{a} | 3.6±0.2^{a} | 4.7±0.4^{b} |

| | | | | | |
|---|---|---|---|---|---|
| 1. Each value is expressed as mean ± SD (n=6). 2. The values marked ^{a,b,c} represent that the stationary platform quadrant is significantly different from other quadrants (p < 0.05). | | | | | |

**Table 8, the retention time in each quadrant and total distance moved on the second day of the working memory test in rats administered GL and SC orally or by injection at different dosages.**

| Total distance moved (cm) | | Quadrant | | | |
|---|---|---|---|---|---|
| | | First Quadrant | Second Quadrant | Third Quadrant | Fourth Quadrant |
| | | Time (s) | | | |
| C | 655.2±35.4 | 8.4±0.9^{a} | 8.2±0.1^{a} | 9.4±0.6^{a} | 9.1±0.1^{a} |
| A | 653.2±29.0 | 7.7±2.7^{a} | 9.6±1.7^{b} | 10.2±1.8^{b} | 11.7±3.0^{b} |
| AM | 564.3±24.6 | 8.5±0.1^{a} | 10.8±0.2^{c} | 9.2±0.4^{a} | 9.2±0.5^{b} |
| GLI1 | 554.2±20.5 | 6.3±0.1^{a} | 9.2±1.0^{b} | 6.6±0.2^{a} | 7.3±0.9^{a} |
| GL1 | 544.2±15.6 | 6.2±0.2^{a} | 8.9±0.4^{b} | 5.5±1.1^{a} | 6.6±1.1^{a} |
| GL5 | 542.2±19.5 | 4.9±0.3^{a} | 8.5±0.2^{b} | 4.3±0.5^{a} | 5.0±0.8^{a} |
| SCI1 | 548.4±21.5 | 6.5±0.2^{a} | 8.7±0.7^{b} | 6.6±0.2^{a} | 7.6±0.2^{a} |
| SC1 | 514.2±14.9 | 6.6±0.3^{a} | 8.3±0.5^{b} | 5.3±0.5^{a} | 6.7±0.1^{a} |
| SC5 | 502.2±12.2 | 4.6±0.1^{a} | 8.5±0.6^{b} | 4.5±0.2^{a} | 5.1±0.3^{a} |

| | | | | | |
|---|---|---|---|---|---|
| 1. Each value is expressed as mean ± SD (n=6). 2. The values marked ^{a,b,c} represent that the stationary platform quadrant is significantly different from other quadrants (p < 0.05). | | | | | |

**Table 9, the retention time in each quadrant and total distance moved on the third day of the working memory test in rats administered GL and SC orally or by injection at different dosages.**

| Total distance moved (cm) | | Quadrant | | | |
|---|---|---|---|---|---|
| | | First Quadrant | Second Quadrant | Third Quadrant | Fourth Quadrant |
| | | Time (s) | | | |
| C | 550.6±32.7 | 5.6±0.8^{a} | 5.3±0.5^{a} | 7.5±2.1^{a} | 6.6±1.3^{a} |
| A | 692.6±23.6 | 6.1±0.3^{a} | 7.5±0.7^{a} | 7.2±0.5^{a} | 7.3±1.0^{a} |
| AM | 521.7±32.6 | 5.5±0.8^{a} | 6.6±0.8^{a} | 9.7±0.2^{b} | 5.7±0.6^{a} |
| GLI1 | 515.8±18.8 | 5.6±0.8^{b} | 4.3±0.7^{a} | 8.4±0.4^{c} | 4.6±0.8^{a} |
| GL1 | 531.9±20.3 | 5.5±0.7^{b} | 4.6±0.3^{a} | 8.1±0.7^{c} | 3.9±0.4^{a} |
| GL5 | 421.9±12.3 | 3.2±0.6^{a} | 2.5±0.8^{a} | 9.8±0.4^{b} | 2.9±0.2^{a} |
| SCI1 | 545.6±19.4 | 5.7±0.3^{a} | 4.6±0.7^{a} | 7.4±0.4^{b} | 4.8±0.4^{a} |
| SC1 | 564.7±25.3 | 5.6±0.3^{a} | 4.4±0.3^{a} | 9.1±0.6^{b} | 3.7±0.3^{a} |
| SC5 | 451.4±12.6 | 3.6±0.4^{a} | 4.0±0.5^{a} | 9.5±0.3^{b} | 3.1±0.5^{a} |

| | | | | | |
|---|---|---|---|---|---|
| 1. Each value is expressed as mean ± SD (n=6). 2. The values marked ^{a,b,c} represent that the stationary platform quadrant is significantly different from other quadrants (p < 0.05). | | | | | |

### Example 4: Effects of purified mushroom β-glucan on acetylcholinesterase activity and oxidative stress in rats

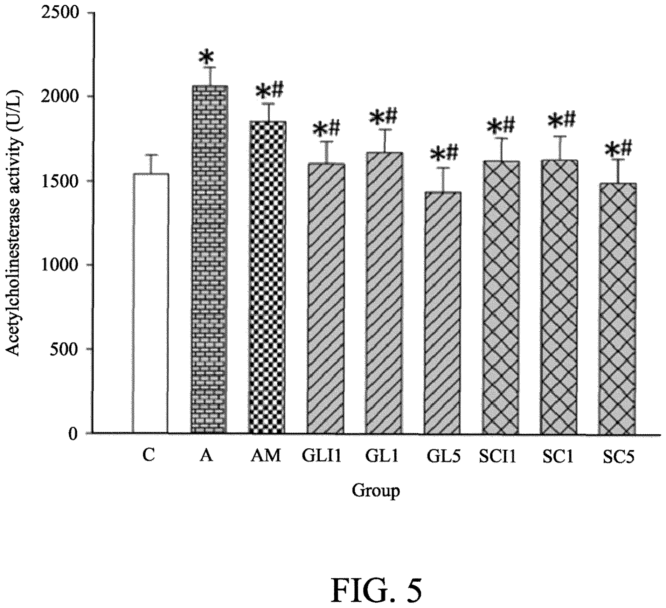

In rats induced by Alzheimer's disease, acetylcholinesterase activity in the body significantly increases due to damaged brain cells and disordered nerve conduction. Therefore, acetylcholinesterase activity in the tested rats was analyzed 28 days after the operation. As shown in Fig. 5, it can be found that the acetylcholinesterase activity in rats administered purified *Ganoderma lucidum* or *Schizophyllum commune* β-glucan orally or by injected was not only lower than that of the No-treatment control group, but also tended to approach the drug control group as the dosage increased. These results show that purified mushroom β-glucan has the function of protecting neurons, and there is no statistical significance between the test group and the AM group. Each value in the diagram is expressed as the mean ± SD (n=6); # indicates statistical significance (p<0.05) compared with the β-amyloid injection group (A Group); * indicates statistical significance (p<0.05) compared with the normal group (C Group).

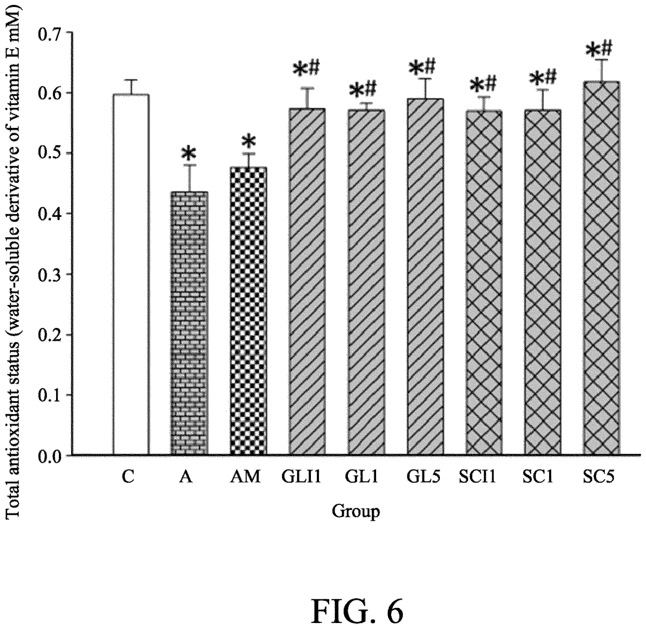

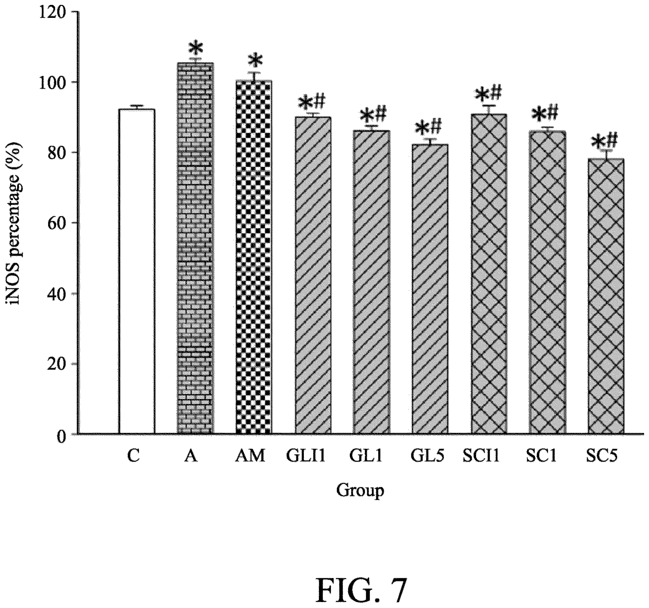

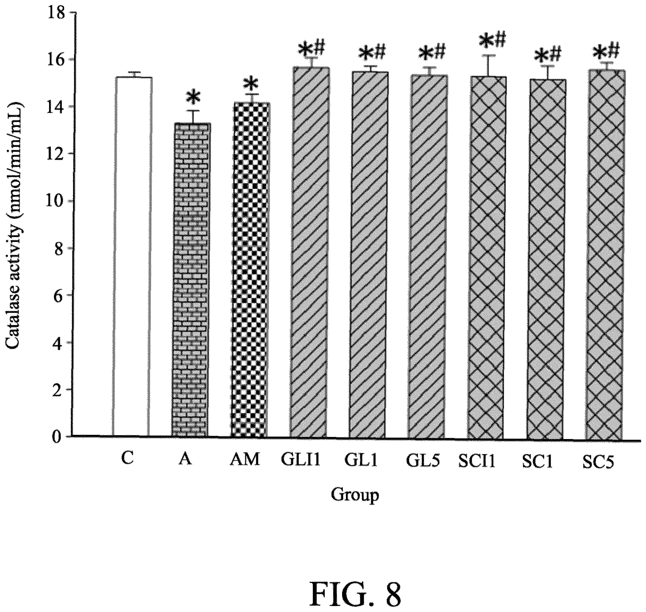

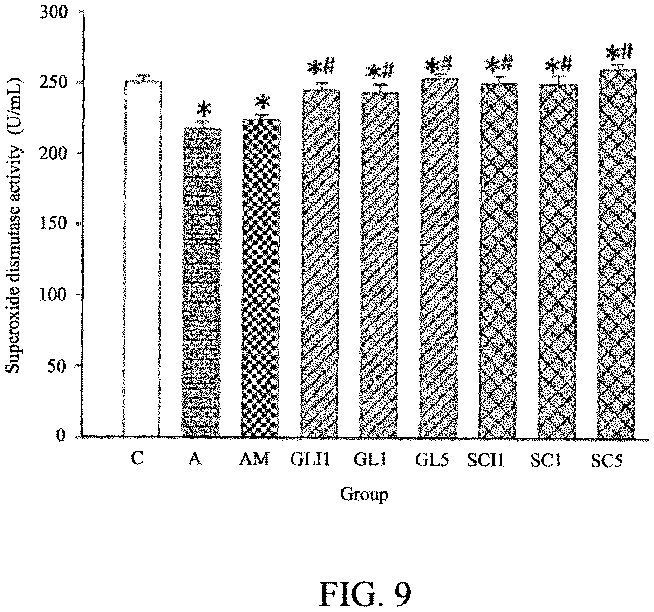

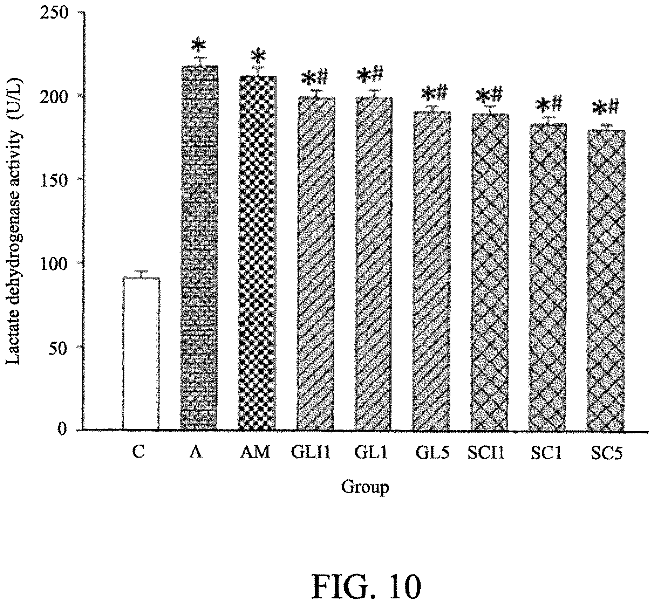

In addition, brain neurons damages due to the increase in oxidative stress and the toxicity of long-term accumulation of oxidative stress. Therefore, blood was drawn from the test rats 28 days after the operation to analyze the total antioxidant capacity and the ability to remove nitric oxide in the body, as well as the changes in activity of catalase, superoxide dismutase and lactate dehydrogenase. As shown in FIGs 6 to 10, it can be found that rats administered purified *Ganoderma lucidum* or *Schizophyllum commune* β-glucan orally or by injection were evaluated using Trolox equivalent antioxidant capacity as the standard for measuring antioxidant capacity. The total antioxidant capacity was significantly improved; the level of inducible nitric oxide synthase (iNOS), which increases the level of nitric oxide in the body, decreased (which means that the ability to remove nitric oxide is improved); the activity of catalase and superoxide dismutase, which have antioxidant functions, increased; and the activity of lactate dehydrogenase, which indicates the degree of cell damage in the body, decreased. These results show that purified mushroom β-glucan has excellent efficacy in reducing oxidative stress. Each test result presented statistical significance between the test groups and the AM group. Each value in the diagram is expressed as the mean ± SD (n=6); # indicates statistical significance (p<0.05) compared with the β-amyloid injection group (Group A); * indicates statistical significance (p<0.05) compared with the normal group (Group C).

### Example 5: Safety assessment of purified mushroom β-glucan

In order to assess the safety of purified mushroom β-glucan, the physiological indicators of the test rats were detected.

The test rats were fed or injected with purified mushroom β-glucan for four weeks after the operation, and the body weight of all test rats were measured and recorded every week. The results showed that purified mushroom beta-glucan did not have a significant effect on the body weight of rats (Table 10).

**Table 10, weekly average body weight changes in rats treated with oral or injected GL and SC at different dosages.**

| | Week 0 | Week 1 | Week 2 | Week 3 | Week 4 |
|---|---|---|---|---|---|
| | Weight (g) | | | | |
| C | 309.3±6.3 | 326.3±4.3 | 345.2±2.3 | 367.0±6.8 | 398.2±3.7 |
| A | 303.6±5.0 | 325.2±4.6 | 342.6±3.0 | 365.7±2.0 | 401.3±3.2 |
| AM | 305.3±5.2 | 322.5±3.5 | 341.2±1.7 | 368.2±4.1 | 408.1±5.2 |
| GLI1 | 306.2±5.3 | 327.3±3.2 | 346.3±2.3 | 367.6±3.8 | 402.2±4.1 |
| GL1 | 305.5±5.0 | 324.2±4.2 | 343.2±2.1 | 365.9±2.6 | 407.4±3.6 |
| GL5 | 304.6±4.2 | 326.4±2.5 | 337.2±1.8 | 367.8±5.4 | 406.4±4.1 |
| SCI1 | 311.2±2.4 | 321.1±4.3 | 346.3±2.5 | 366.9±3.2 | 404.2±5.4 |
| SC1 | 304.4±3.2 | 323.5±5.3 | 346.2±3.2 | 366.7±3.3 | 411.4±4.2 |
| SC5 | 307.8±2.0 | 325.4±5.1 | 346.8±2.0 | 367.7±2.5 | 412.3±5.4 |

| | | | | | |
|---|---|---|---|---|---|
| Each value is expressed as mean ± SD (n=6). | | | | | |

On the 28th day after the operation, the total cholesterol, triglycerides, high-density lipoprotein cholesterol, low-density lipoprotein cholesterol and creatine kinase in the blood of all the test rats were analyzed, and the results showed that there was no statistical significance in relevant indicators in all the test rats (as shown in Table 11).

**Table 11, changes in concentration of cholesterol, triglycerides, high-density lipoprotein cholesterol (HDL-C), low-density lipoprotein cholesterol (LDL-C) and creatine kinase (CPK) in the blood of the rats administered GL and SC orally or by injection at different dosages.**

| | Triglyceride | Cholesterol | HDL-C | LDL-C | CPK |
|---|---|---|---|---|---|
| | (mg/dL) | | | | (U/L) |
| C | 72.6±4.0 | 171.1±3.8 | 47.8±4.5 | 146.8±2.6 | 214.7±21.2 |
| A | 70.3±4.2 | 181.3±3.8 | 48.3±7.5 | 141.3±1.4 | 315.1±27.1 |
| AM | 70.6±4.6 | 171.5±3.6 | 56.4±5.0 | 121.0±2.3 | 332.3±20.4 |
| GLI1 | 72.7±4.0 | 172.6±1.1 | 60.4±6.0 | 140.6±3.6 | 322.2±34.4 |
| GL1 | 71.0±4.2 | 178.2±3.2 | 59.5±6.3 | 139.5±2.3 | 322.1±29.3 |
| GL5 | 69.6±6.7 | 179.3±2.5 | 47.8±8.2 | 137.9±3.1 | 319.5±29.2 |
| SCI1 | 72.7±4.0 | 173.8±2.1 | 58.4±8.0 | 142.7±1.6 | 292.3±24.4 |
| SC1 | 71.0±4.2 | 171.3±4.3 | 56.3±7.3 | 141.5±2.6 | 299.4±25.3 |
| SC5 | 69.6±6.7 | 171.4±2.6 | 49.8±8.5 | 139.6±3.6 | 316.3±31.2 |

| | | | | | |
|---|---|---|---|---|---|
| Each value is expressed as mean ± SD (n=6). | | | | | |

On the 28th day after the operation, the aspartate aminotransferase, alanine aminotransferase, alkaline phosphatase, total protein, albumin and globulin in the blood of all the test rats were analyzed, and the results showed that there was no statistical significance in relevant indicators in all the test rats (as shown in Table 12).

**Table 12, changes in aspartate transaminase (AST), alanine transaminase (ALT), alkaline phosphatase (Alk-p), total protein, albumin and globulin in the blood of the rats administered GL and SC orally or by injection at different dosages.**

| | AST | ALT | Alk-p | Total Protein | Albumin | Globulin |
|---|---|---|---|---|---|---|
| | (U/L) | | (IU/L) | (g/dL) | | |
| C | 112.2±6.9 | 46.7±3.6 | 125.5±9.8 | 6.9±0.3 | 4.6±0.2 | 2.3±0.2 |
| A | 115.1±5.6 | 44.2±4.0 | 129.5±6.6 | 7.1±0.3 | 4.8±0.2 | 2.3±0.3 |
| AM | 113.5±2.1 | 45.6±3.3 | 130.5± 4.9 | 7.1±0.2 | 4.7±0.2 | 2.4±0.2 |
| GLI1 | 115.3±1.6 | 46.5±0.9 | 136.2±2.8 | 7.0±0.3 | 4.6±0.2 | 2.3±0.3 |
| GL1 | 111.7±4.3 | 45.3±6.0 | 134.3±2.3 | 7.0±0.4 | 4.5±0.3 | 2.5±0.3 |
| GL5 | 111.4±3.4 | 45.1±4.3 | 129.0±3.0 | 7.0±0.5 | 4.7±0.4 | 2.2±0.2 |
| SCI1 | 113.3±2.6 | 45.8±1.9 | 137.2±3.3 | 7.1±0.2 | 4.6±0.4 | 2.4±0.2 |
| SC1 | 116.7±4.2 | 45.9±3.0 | 136.3±3.6 | 7.0±0.3 | 4.6±0.2 | 2.3±0.2 |
| SC5 | 116.4±5.7 | 45.7±3.3 | 140.0±4.3 | 7.0±0.3 | 4.7±0.3 | 2.4±0.3 |

| | | | | | | |
|---|---|---|---|---|---|---|
| Each value is expressed as mean ± SD (n=6). | | | | | | |

On the 28th day after the operation, the renal function indicators including urea nitrogen, creatinine and uric acid in the blood of all the test rats were analyzed, and the results showed that there was no statistical significance in relevant indicators in all the test rats (as shown in Table 13).

**Table 13, changes in renal function indicators including urea nitrogen (BUN), creatinine and uric acid in the blood of the rats administered GL and SC orally or by injection at different dosages.**

| | BUN | Creatinine | Uric Acid |
|---|---|---|---|
| | | (mg/dL) | |
| C | 16.8±1.7 | 0.46±0.06 | 6.6±1.2 |
| A | 18.4±2.6 | 0.49±0.06 | 6.5±1.3 |
| AM | 23.1±3.8 | 0.46±0.07 | 5.6±1.3 |
| GLI1 | 21.6±3.7 | 0.45±0.06 | 5.8±1.2 |
| GL1 | 19.6±4.9 | 0.52±0.08 | 5.6±1.3 |
| GL5 | 18.4±2.4 | 0.48±0.07 | 6.5±1.1 |
| SCI1 | 22.7±2.6 | 0.48±0.08 | 5.9±1.4 |
| SC1 | 21.6±1.9 | 0.51±0.05 | 5.6±1.1 |
| SC5 | 20.4±2.4 | 0.49±0.04 | 6.1±1.2 |

| | | | |
|---|---|---|---|
| Each value is expressed as mean ± SD (n=6). | | | |

On the 28th day after the operation, the electrolytes including sodium, potassium, chlorine, calcium and phosphorus in the blood of all the test rats were analyzed, and the results showed that there was no statistical significance in relevant indicators in all the test rats (as shown in Table 14).

**Table 14, changes in electrolytes including sodium, potassium, chlorine, calcium and phosphorus in the blood of rats administered GL and SC orally or by injection at different dosages.**

| | Sodium | Potassium | Chlorine | Calcium | Phosphorus |
|---|---|---|---|---|---|
| | mEq/L | | | mg/dL | |
| C | 147.5±2.7 | 8.6±1.9 | 94.5±2.1 | 12.4±0.7 | 13.6±1.3 |
| A | 148.7±1.5 | 7.4±0.7 | 93.6±1.4 | 12.6±0.7 | 14.5±1.6 |
| AM | 148.9±1.4 | 7.2±0.9 | 93.9±2.3 | 12.6±0.8 | 13.9±2.3 |
| GLI1 | 150.2±1.1 | 7.5±0.9 | 91.6±1.6 | 12.4±0.5 | 14.4±2.6 |
| GL1 | 148.8±1.9 | 7.3±0.6 | 92.3±1.6 | 12.5±0.7 | 15.4±1.6 |
| GL5 | 148.6±1.6 | 7.1±0.8 | 93.3±1.7 | 12.4±0.6 | 14.1±2.4 |
| SCI1 | 146.3±1.2 | 7.3±0.6 | 93.1±1.3 | 12.3±0.6 | 14.0±1.6 |
| SC1 | 147.1±1.4 | 7.2±0.4 | 92.4±1.5 | 12.5±0.5 | 15.2±2.6 |
| SC5 | 149.2± 1.3 | 7.4±0.5 | 92.6±1.5 | 12.2±0.5 | 14.7±1.4 |

| | | | | | |
|---|---|---|---|---|---|
| Each value is expressed as mean ± SD (n=6). | | | | | |

It can be seen from the above results that the purified mushroom β-glucan extracted from *Ganoderma lucidum* or *Schizophyllum commune* do not have a significant impact on the physiological functions and blood indicators of rats, so it is confirmed that the purified mushroom β-glucan of the present disclosure has excellent safety for animals and can be further developed into food or drugs.

Further embodiments of the invention include:
1. A method for preventing, improving or treating Alzheimer's disease, dementia or brain function degeneration in a subject in need, comprising administering to the subject a composition comprising purified mushroom β-glucan, wherein the purified mushroom β-glucan is derived from mushroom mycelium or its fermentation product, wherein the purity of the purified mushroom β-glucan is 60% or above.
2. The method of embodiment 1, wherein a mushroom of the mushroom mycelium is at least one selected from the group consisting of *Ganoderma lucidum*, *Schizophyllum commune*, *Agaricus blazei*, *Cordyceps sinensis*, *Coriolus versicolor*, *Taiwanofungus camphoratus*, *Phellinus linteus*, *Hericium erinaceus*, *Auricularia auricula* and *Cordyceps militaris.*
3. The method of embodiment 1, wherein the prevention, improvement or treatment of Alzheimer's disease, dementia or brain function degeneration comprises increasing the viability of neurons by the purified mushroom β-glucan.
4. The method of embodiment 1, comprising decreasing oxidative stress of neurons by the purified mushroom β-glucan.
5. The method of embodiment 4, wherein reducing oxidative stress of neurons comprises at least one of decreasing the level of inducible nitric oxide synthase, increasing the activity of catalase and/or superoxide dismutase, and decreasing the activity of lactate dehydrogenase in the subject.
6. The method of embodiment 1, comprising decreasing the cytotoxicity to neurons caused by amyloid (amyloid β (1-40)) and/or decreasing amyloid accumulation by the purified mushroom β-glucan.
7. The method of embodiment 1, comprising preventing, improving or treating cognitive dysfunction by the purified mushroom β-glucan, wherein the cognitive dysfunction comprises at least one of memory impairment, memory loss, learning impairment and loss of spatial perception.
8. The method of embodiment 1, wherein the purified mushroom β-glucan is obtained by the following steps, comprising:
   taking fermented mushroom mycelium and fermentation broth after liquid fermentation and then obtaining crude extract after ultrasonic disruption;
   centrifuging the crude extract and taking the supernatant;
   performing a first filtration on the supernatant with a 0.8 to 3 µm filter membrane and then a 0.22 to 0.5 µm filter membrane to filter the supernatant;
   performing a first freeze-drying to prepare mushroom polysaccharide raw material;
   redissolving the mushroom polysaccharide raw material in water to form a redissolved liquid;
   performing a second filtration on the redissolved liquid with an ultrafiltration ceramic membrane filter column with a molecular weight cutoff (MWCO) from 80 to 150KD and then a MWCO from 2 to 10KD to remove impurities;
   performing a third filtration with a 0.22 µm filter membrane; and
   performing a second freeze-drying to obtain the purified mushroom β -glucan.
9. The method of embodiment 1, wherein the composition is in the form of powder, tablet, suppository, microcapsule, liquid, spray or insert.
10.The method of embodiment 1, wherein the composition further comprises excipients.
11. The method of embodiment 10, wherein the composition is obtained by mixing the purified mushroom β-glucan with excipients and then drying.
12.The method of embodiment 10, wherein the excipient is at least one selected from the group consisting of lactose, sucrose, glucose, fructooligosaccharides, starch, starch derivative and dietary fiber.
13.The method of embodiment 9, wherein the composition is obtained by mixing the purified mushroom β-glucan with a solvent or buffer.
14.The method of embodiment 1, wherein the composition is a pharmaceutical, feed, feed additive, beverage, nutritional supplement, dairy product, food, food additive, health food raw material or health food finished product.
15.The method of embodiment 1, wherein the composition further comprises an additive, wherein the additive is at least one selected from the group consisting of a carrier, preservative, diluent, filler, absorption enhancer, sweetener, and adjuvant.
16.The method of embodiment 14, wherein the composition is a pharmaceutical, comprising the purified mushroom β-glucan and its pharmaceutically acceptable carrier.
17.The method of embodiment 16, wherein the pharmaceutical is administered to the subject at an effective amount of 0.04 to 1 mg/kg.bw.
18.The method of embodiment 16, wherein the pharmaceutical is administered orally, intrarectally, transdermally, transmucosally or by injection.

## Claims

1. A composition comprising purified mushroom β-glucan for use in preventing, improving or treating Alzheimer's disease, dementia or brain function degeneration in a subject in need thereof, wherein the purified mushroom β-glucan is derived from mushroom mycelium or its fermentation product, wherein the purity of the purified mushroom β-glucan is 60% or above.

2. The composition for use according to claim 1, wherein a mushroom of the mushroom mycelium is at least one selected from the group consisting of *Ganoderma lucidum*, *Schizophyllum commune*, *Agaricus blazei*, *Cordyceps sinensis*, *Coriolus versicolor*, *Taiwanofungus camphoratus*, *Phellinus linteus*, *Hericium erinaceus*, *Auricularia auricula* and *Cordyceps militaris.*

3. The composition for use according to claim 1 or claim 2, wherein the prevention, improvement or treatment of Alzheimer's disease, dementia or brain function degeneration comprises increasing the viability of neurons by the purified mushroom β-glucan.

4. The composition for use according to any preceding claim, wherein the prevention, improvement or treatment of Alzheimer's disease, dementia or brain function degeneration comprises decreasing oxidative stress of neurons by the purified mushroom β-glucan, preferably decreasing oxidative stress of neurons comprises at least one of decreasing the level of inducible nitric oxide synthase, increasing the activity of catalase and/or superoxide dismutase, and decreasing the activity of lactate dehydrogenase in the subject.

5. The composition for use according to any preceding claim, wherein the prevention, improvement or treatment of Alzheimer's disease, dementia or brain function degeneration comprises decreasing the cytotoxicity to neurons caused by amyloid (amyloid β (1-40)) and/or decreasing amyloid accumulation by the purified mushroom β-glucan.

6. The composition for use according to any preceding claim, wherein the prevention, improvement or treatment of Alzheimer's disease, dementia or brain function degeneration comprises preventing, improving or treating cognitive dysfunction by the purified mushroom β-glucan, wherein the cognitive dysfunction comprises at least one of memory impairment, memory loss, learning impairment and loss of spatial perception.

7. The composition for use according to any preceding claim, wherein the purified mushroom β-glucan is obtained by the following steps, comprising:
taking fermented mushroom mycelium and fermentation broth after liquid fermentation and then obtaining crude extract after ultrasonic disruption;
centrifuging the crude extract and taking the supernatant;
performing a first filtration on the supernatant with a 0.8 to 3 µm filter membrane and then a 0.22 to 0.5 µm filter membrane to filter the supernatant;
performing a first freeze-drying to prepare mushroom polysaccharide raw material;
redissolving the mushroom polysaccharide raw material in water to form a redissolved liquid;
performing a second filtration on the redissolved liquid with an ultrafiltration ceramic membrane filter column with a molecular weight cutoff (MWCO) from 80 to 150KD and then a MWCO from 2 to 10KD to remove impurities;
performing a third filtration with a 0.22 µm filter membrane; and
performing a second freeze-drying to obtain the purified mushroom β - glucan.

8. The composition for use according to any preceding claim, wherein the composition is in the form of powder, tablet, suppository, microcapsule, liquid, spray or insert.

9. The composition for use according to any preceding claim, wherein the composition further comprises one or more excipients, preferably the excipients are selected from the group consisting of lactose, sucrose, glucose, fructooligosaccharides, starch, starch derivative and dietary fiber, and preferably wherein the composition is obtained by mixing the purified mushroom β-glucan with the excipients and then drying.

10. The composition for use according to claim 8, wherein the composition is obtained by mixing the purified mushroom β-glucan with a solvent or buffer.

11. The composition for use according to any preceding claim, wherein the composition is a pharmaceutical, feed, feed additive, beverage, nutritional supplement, dairy product, food, food additive, health food raw material or health food finished product.

12. The composition for use according to claim 11, wherein the composition is a pharmaceutical comprising the purified mushroom β-glucan and its pharmaceutically acceptable carrier.

13. The composition for use according to any preceding claim, wherein the composition further comprises an additive, wherein the additive is at least one selected from the group consisting of a carrier, preservative, diluent, filler, absorption enhancer, sweetener, and adjuvant.

14. The composition for use according to claims 11 or 12, wherein the pharmaceutical is administered to the subject at an effective amount of 0.04 to 1 mg/kg.bw.

15. The composition for use according to claims 11 or 12, wherein the pharmaceutical is administered orally, intrarectally, transdermally, transmucosally or by injection.
